(19) Europäisches Patentamt
European Patent Office
Office européen des brevets

(11) Veröffentlichungsnummer: **0 340 169 B1**

# (12) EUROPÄISCHE PATENTSCHRIFT

(45) Veröffentlichungstag der Patentschrift: **30.11.94**

(51) Int. Cl.5: **C07D 491/20**, B41M 5/124, C07D 491/00, B41M 5/00, B41M 5/30, //(C07D491/20, 311:00,307:00,231:00)

(21) Anmeldenummer: **89810293.4**

(22) Anmeldetag: **18.04.89**

Die Akte enthält technische Angaben, die nach dem Eingang der Anmeldung eingereicht wurden und die nicht in dieser Patentschrift enthalten sind.

## (54) Chromogene Laktonverbindungen von Benzopyrano-2H-pyrazolen.

(30) Priorität: **27.04.88 CH 1568/88**

(43) Veröffentlichungstag der Anmeldung:
**02.11.89 Patentblatt 89/44**

(45) Bekanntmachung des Hinweises auf die Patenterteilung:
**30.11.94 Patentblatt 94/48**

(84) Benannte Vertragsstaaten:
**AT BE CH DE ES FR GB IT LI**

(56) Entgegenhaltungen:
**FR-A- 2 243 084**
**GB-A- 1 469 516**

(73) Patentinhaber: **CIBA-GEIGY AG**
**Klybeckstrasse 141**
**CH-4002 Basel (CH)**

(72) Erfinder: **Fletcher, Ian John, Dr.**
**Bürgenstal 27**
**CH-4312 Magden (CH)**

EP 0 340 169 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft chromogene Laktonverbindungen von Benzopyrano-2H-pyrazolverbindungen, Verfahren zu ihrer Herstellung und ihre Verwendung als Farbbildner in druckempfindlichen oder wärmeempfindlichen Aufzeichnungsmaterialien.

In den Dokumenten FR-A-2,243,084 und GB-A-1,469,516 werden chromogene Lactone von Benzopyrano-2H-pyrazolderivaten offenbart, die sich von den erfindungsgemässen Lactonverbindungen durch die Position des Arylsubstituenten am Stickstoffatom des Pyrazolringes unterscheiden.

Die erfindungsgemässen Laktonverbindungen von Benzopyrano-2H-pyrazolen entsprechen der allgemeinen Formel

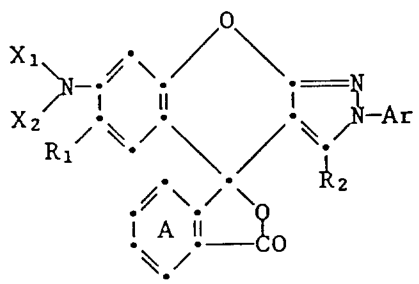

(1)

worin

| | |
|---|---|
| Ar | einen unsubstituierten oder durch Halogen, Nitro, Cyano, $C_1$-$C_5$-Alkyl, $C_1$-$C_5$-Alkoxy, $C_1$-$C_5$-Alkylthio, $C_1$-$C_5$-Alkoxycarbonyl, Trifluormethyl, Phenoxy, Phenylthio oder -$NX_3X_4$ substituierten Diphenyl-, Naphthyl- oder Phenylrest, |
| $R_1$ | Wasserstoff, Halogen, $C_1$-$C_5$-Alkyl oder $C_1$-$C_5$-Alkoxy, |
| $R_2$ | $C_1$-$C_5$-Alkyl, Phenyl oder durch Halogen, $C_1$-$C_5$-Alkyl oder $C_1$-$C_5$-Alkoxy substituiertes Phenyl, und |
| $X_1$, $X_2$, $X_3$ und $X_4$, | unabhängig voneinander, je Wasserstoff, unsubstituiertes oder durch Halogen, Hydroxy, Cyano, Tetrahydrofuryl oder $C_1$-$C_5$-Alkoxy substituiertes Alkyl mit höchstens 12 Kohlenstoffatomen, Acyl mit 1 bis 12 Kohlenstoffatomen, Cycloalkyl mit 5 bis 10 Kohlenstoffatomen oder unsubstituiertes oder durch Halogen, Cyano, Nitro, Trifluormethyl, $C_1$-$C_5$-Alkyl, $C_1$-$C_5$-Alkoxy oder $C_1$-$C_5$-Alkoxycarbonyl substituiertes Phenethyl, Phenylisopropyl oder Benzyl oder einen Diphenyl-, Naphthyl- oder Phenylrest, oder die Substituentenpaare ($X_1$ und $X_2$) und ($X_3$ und $X_4$) je zusammen mit dem gemeinsamen Stickstoffatom einen fünf- oder sechsgliedrigen, vorzugsweise gesättigten, Pyrrolidino-, Piperidino-, Pipecolino-, Morpholino-, Thiomorpholino- oder Piperazinrest bedeuten und worin |

der Ring A unsubstituiert oder durch Halogen, Nitro, $C_1$-$C_5$-Alkyl, $C_1$-$C_5$-Alkoxy, $C_1$-$C_5$-Alkylthio, $C_1$-$C_5$-Alkoxycarbonyl, Amino, Mono-$C_1$-$C_5$-Alkylamino oder Di-$C_1$-$C_5$-Alkylamino substituiert ist.

$C_1$-$C_5$-Alkyl, $C_1$-$C_5$-Alkoxy und $C_1$-$C_5$-Alkylthio stellen bei der Definition der Reste der Laktonverbindungen solche Gruppen oder Gruppenbestandteile dar, die 1 bis 5, insbesondere 1 bis 3 Kohlenstoffatome aufweisen. Beispiele für derartige Gruppen sind Methyl, Ethyl, n-Propyl, Isopropyl, n-Butyl, sek.-Butyl, tert.Butyl, Amyl, Isoamyl oder tert.Amyl bzw. Methoxy, Ethoxy, Isopropoxy, Isobutoxy oder tert.Butoxy bzw. Methylthio, Ethylthio, Propylthio oder Butylthio.

Halogen bedeutet beispielsweise Fluor, Brom oder vorzugsweise Chlor.

Acyl ist besonders Formyl, $C_1$-$C_5$-Alkylcarbonyl, wie z.B. Acetyl oder Propionyl, oder Benzoyl. Weitere Acylreste können $C_1$-$C_5$-Alkylsulfonyl, wie z.B. Methylsulfonyl oder Ethylsulfonyl sowie Phenylsulfonyl sein. Benzoyl und Phenylsulfonyl können durch Halogen, Methyl, Methoxy oder Ethoxy substituiert sein.

$R_1$ ist vorzugsweise Methyl, Methoxy, Brom, Chlor oder vor allem Wasserstoff.

$R_2$ ist vorzugsweise $C_1$-$C_5$-Alkyl und besonders Methyl. Als gegebenenfalls substituiertes Phenyl kann $R_2$ Phenyl, Tolyl, Xylyl, Chlorphenyl, Methoxyphenyl oder Carbomethoxyphenyl darstellen.

Vorzugsweise bedeutet Ar einen unsubstituierten oder durch Halogen, Nitro, $C_1$-$C_5$-Alkyl, $C_1$-$C_5$-Alkoxy, $C_1$-$C_5$-Alkoxycarbonyl, Phenoxy oder -$NX_3X_4$ substituierten Phenyl- oder Naphthylrest.

Stellen die Substituenten $X_1$, $X_2$, $X_3$ und $X_4$ Alkylgruppen dar, so können sie geradkettig oder verzweigt sein. Beispiele solcher Alkylreste sind Methyl, Ethyl, n-Propyl, Isopropyl, n-Butyl, sek.-Butyl, tert.-Butyl, Amyl, Isoamyl, n-Hexyl, 2-Ethyl-hexyl, n-Heptyl, n-Octyl, Isooctyl, n-Nonyl, Isononyl oder n-Dodecyl.

Sind die Alkylreste in $X_1$, $X_2$, $X_3$ und $X_4$ substituiert, so handelt es sich vor allem um Cyanoalkyl, Halogenalkyl, Hydroxyalkyl, Alkoxyalkyl jeweils vorzugsweise mit insgesamt 2 bis 6 Kohlenstoffatomen, wie z.B. $\beta$-Cyanoethyl, $\beta$-Chlorethyl, $\gamma$-Chlorpropyl, $\beta$-Hydroxyethyl, $\gamma$-Hydroxypropyl, $\beta$-Methoxyethyl, $\beta$-Ethoxyethyl oder $\gamma$-Methoxypropyl sowie auch Tetrahydrofurfuryl.

Beispiele für Cycloalkyl in der Bedeutung der X-Reste sind Cyclopentyl, Cycloheptyl oder vorzugsweise Cyclohexyl. Die Cycloalkylreste können einen oder mehrere $C_1$-$C_4$-Alkylreste, vorzugsweise Methylgruppen, enthalten und weisen insgesamt 5 bis 10 Kohlenstoffatome auf.

Als Diphenyl-, Naphthyl- oder Phenylreste stellen die X-Reste besonders Naphthyl oder in erster Linie Phenyl dar.

Bevorzugte Substituenten in der Phenethyl-, Phenylisopropyl- oder Benzyl- und Diphenyl-, Naphthyl- oder Phenyl-gruppe der X-Reste sind z.B. Halogen, Cyano, Methyl, Trifluormethyl, Methoxy oder Carbomethoxy. Beispiele für derartige araliphatische bzw. aromatische Reste sind Methylbenzyl, 2,4- oder 2,5-Dimethylbenzyl, Chlorbenzyl, Dichlorbenzyl, Cyanobenzyl, Tolyl, Xylyl, Chlorphenyl, Methoxyphenyl, Trifluormethylphenyl, 2,6-Dimethylphenyl oder Carbomethoxyphenyl.

Bevorzugte gesättigte heterocyclische Reste für $-NX_1X_2$ und $-NX_3X_4$ sind Pyrrolidino, Piperidino oder Morpholino.

Die Substituenten $X_1$ und $X_2$ sind vorzugsweise Cyclohexyl, Tolyl, Benzyl, Cyano-$C_1$-$C_5$-Alkyl z.B. $\beta$-Cyanoethyl oder in erster Linie $C_1$-$C_5$-Alkyl, wie z.B. Methyl, Ethyl, n-Propyl, Isopropyl, n-Butyl oder Isoamyl. $-NX_1X_2$ ist bevorzugt auch Pyrrolidinyl oder N-$C_1$-$C_5$-Alkyl-N-tetrahydrofurfurylamino.

Die Substituenten $X_3$ und $X_4$ sind vorzugsweise Wasserstoff, $C_1$-$C_8$-Alkyl, Cyclohexyl, Phenyl, Benzyl oder durch Halogen, Methyl, Methoxy, Carbomethoxy oder Trifluormethyl substituiertes Phenyl oder Benzyl. $-NX_3X_4$ ist vorzugsweise auch Acetylamino, Propionylamino oder Benzoylamino.

Der Ring A ist vorteilhafterweise nicht weiter substituiert. Falls er Substituenten aufweist, ist er in erster Linie durch Halogen, Nitro, $C_1$-$C_5$-Alkyl, $C_1$-$C_5$-Alkoxycarbonyl oder Di-$C_1$-$C_5$-Alkylamino substituiert. Der Ring A ist bevorzugt unsubstituiert oder durch Halogen substituiert.

Praktisch wichtige Laktonverbindungen von Benzopyrano-2H-pyrazolen entsprechen der Formel

(2) 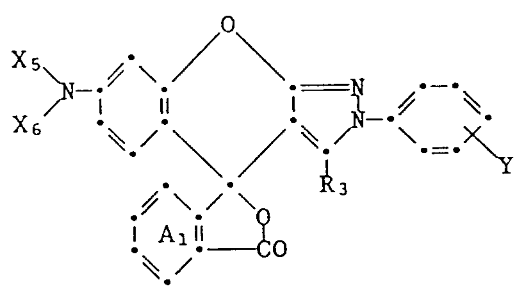

worin

$R_3$ $C_1$-$C_5$-Alkyl oder Phenyl,

Y Wasserstoff, Halogen, Nitro, $C_1$-$C_5$-Alkyl, $C_1$-$C_5$-Alkoxy, Phenoxy oder $-NX_7X_8$,

$X_8$, $X_8$, $X_7$ und $X_8$, unabhängig voneinander, je $C_1$-$C_8$-Alkyl, $C_5$-$C_6$-Cycloalkyl oder unsubstituiertes oder durch Halogen, $C_1$-$C_5$-Alkyl, $C_1$-$C_5$-Alkoxy oder $C_1$-$C_5$-Alkoxycarbonyl substituiertes Benzyl oder Phenyl und $X_8$ auch Wasserstoff oder

die Substituentenpaare ($X_8$ und $X_8$) und ($X_7$ und $X_8$), unabhängig voneinander, je zusammen mit dem jeweiligen gemeinsamen Stickstoffatom Pyrrolidino, Piperidino oder Morpholino bedeuten und der Ring $A_1$ unsubstituiert oder durch Halogen, $C_1$-$C_5$-Alkyl, $C_1$-$C_5$-Alkoxycarbonyl oder Di-$C_1$-$C_5$-Alkylamino substituiert ist.

Unter den Laktonverbindungen der Formel (2) sind diejenigen, in denen

$R_3$ $C_1$-$C_5$-Alkyl, insbesondere Methyl und

Y Wasserstoff, Halogen, Nitro, Methyl oder Di-$C_1$-$C_5$-Alkylamino bedeuten und der Ring $A_1$ unsubstituiert ist, besonders bevorzugt.

Von besonderem Interesse sind Laktonverbindungen von Benzopyrano-2H-pyrazolen der Formel

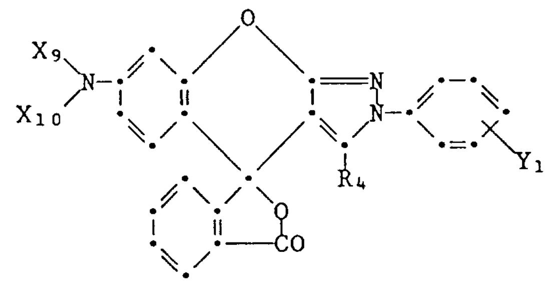

(3)

worin

- $R_4$ Methyl oder Phenyl,
- $Y_1$ Wasserstoff, Chlor, Nitro, Methyl, Carbomethoxy oder Di-$C_1$-$C_5$-Alkylamino,
- $X_9$ $C_1$-$C_6$-Alkyl, Cyclohexyl, Benzyl, Phenyl oder durch Methyl oder Chlor substituiertes Phenyl und
- $X_{10}$ $C_1$-$C_6$-Alkyl bedeuten.

Ganz besonders bevorzugt sind Laktonverbindungen der Formel (3), in der $X_9$ und $X_{10}$ $C_1$-$C_5$-Alkyl, $R_4$ Methyl und $Y_1$ Wasserstoff, Chlor, Nitro, Methyl oder Dimethylamino bedeuten.

Die erfindungsgemässen Laktonverbindungen der Formeln (1) bis (3) werden dadurch hergestellt, dass man eine Ketosäureverbindung der Formel

(4)

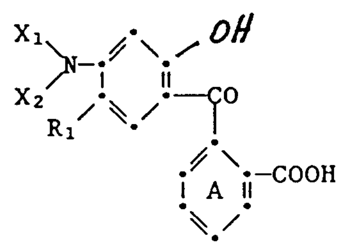

mit einer Pyrazolonverbindung der Formel

(5)

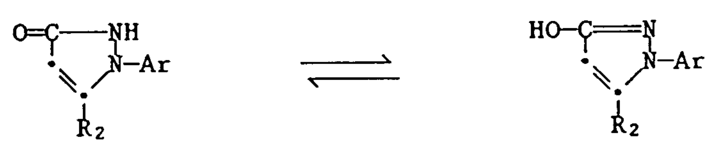

worin

A, Ar, $R_1$, $R_2$, $X_1$ und $X_2$ die angegebene Bedeutung haben, umsetzt.

Die Umsetzung wird zweckmässigerweise so ausgeführt, dass man die Reaktionskomponenten in Gegenwart eines sauren Kondensationsmittels bei einer Temperatur von 20 bis 140 °C zur Reaktion bringt. Beispiele für derartige Kondensationsmittel sind Essigsäureanhydrid, Zinkchlorid, Aluminiumchlorid, Schwefelsäure, Phosphorsäure und Phosphoroxychlorid.

Die Isolierung des Endproduktes der Formel (1) erfolgt in allgemein bekannter Weise durch Einstellen des Reaktionsgemisches auf einen pH-Wert von mindestens 6, vorzugsweise 7 bis 14, z.B. mit Alkalien, wie z.B. Alkalimetallhydroxiden, Ammoniak, Alkalimetallcarbonaten oder -bicarbonaten und Abtrennen des gebildeten Produktes, Waschen und Trocknen oder durch Behandeln mit geeigneten organischen Lösungsmitteln, wie z.B. Methanol, Isopropanol, Ligroin, Benzol, Chlorbenzol, Toluol oder Xylol.

Die Ausgangsstoffe der Formel (4) sind grösstenteils bekannt. Sie werden durch Umsetzung des Säureanhydrids der Formel

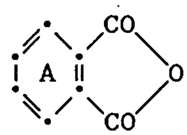

(6)

mit einer Hydroxyverbindung der Formel

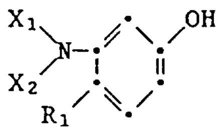

(7)

vorzugsweise in einem organischen Lösungsmittel z.B. Benzol, Toluol, Xylol oder Chlorbenzol erhalten. In Formeln (6) und (7) haben A, $R_1$, $X_1$ und $X_2$ die angegebene Bedeutung.

Die Ausgangsstoffe der Formel (5) sind ebenfalls zum Teil bekannt. Sie können z.B. gemäss C. Venturello, R. D'Aloisio, Synthesis 1979, 283 aus 2-Arylazo-2,5-dimethyl-3-oxo-2,3-dihydrofuranen in stark saurem Medium in Gegenwart von Essigsäure und konz. Salzsäure und unter Bildung der entsprechenden N-Acetylderivate als Zwischenprodukte hergestellt werden. Eine weitere Herstellungsmethode für Ausgangsstoffe der Formel (5) ist in der US-A-2,227,654 beschrieben.

Verbindungen der Formel (1), worin Ar einen durch $-NX_3X_4$ substituierten Diphenyl-, Naphthyl- oder Phenylrest darstellt, können auch dadurch hergestellt werden, dass man eine Verbindung der Formel (1), worin Ar einen Nitrodiphenyl-, Nitronaphthyl- oder Nitrophenylrest bedeutet, auf übliche Weise zur Aminoarylgruppe reduziert und dann mit einem reaktiven Ester eines Alkyl- oder Benzylalkohols und einer anorganischen oder organischen Säure oder auch mit einem reaktiven funktionellen Derivat einer Carbonsäure, insbesondere Fettsäurehalogeniden oder -anhydriden, wie z.B. Acetylchlorid, Acetylbromid oder Acetanhydrid umsetzt. Beispiele für obengenannte reaktive Ester sind Methyl-, Ethyl-, n-Propyl-, n-Butyl- oder Benzylester der Chlorwasserstoff-, Bromwasserstoff- bzw. Jodwasserstoffsäure, Dimethyl- oder Diethylsulfat.

Die Laktonverbindungen von Benzopyrano-2H-pyrazolen der Formeln (1) bis (3) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie je nach Bedeutung von Ar, insbesondere von Y und dem verwendeten Entwickler sofort intensive gelbe, orange oder rote Farbtöne, die besonders licht-und sublimationsecht sind.

Die Laktonverbindungen der Formeln (1) bis (3) sind auch sehr wertvoll im Gemisch mit einem oder mehreren anderen bekannten Farbbildnern, z.B. 3,3-(Bis-aminophenyl-)-phthaliden, 3-Indolyl-3-aminophenylazaphthaliden, (3,3-Bis-indolyl-)-phthaliden, 3-Amino-fluoranen, 3-Dialkylamino-7-dibenzylaminofluoranen, 3-Dialkylamino-6-methyl-7-arylaminofluoranen, Leukoauraminen, Spiropyranen, Spirodipyranen, Chromenoindolen, Phenoxazinen, Phenothiazinen, Chinazolinen, Rhodaminlaktamen, Carbazolylmethanen oder weiteren Triarylmethan-leukofarbstoffen, um graue oder schwarze Färbungen zu erzeugen.

Die Laktonverbindungen der Formeln (1) bis (3) zeigen sowohl auf aktivierten Tonen, wie auch auf phenolischen Unterlagen eine ausgezeichnete Farbintensität. Sie eignen sich insbesondere als sich schnell entwickelnde Farbbildner für die Verwendung in einem wärmeempfindlichen oder insbesondere druckempfindlichen Aufzeichnungsmaterial, das sowohl Kopierals auch Registriermaterial sein kann. Sie zeichnen sich dadurch aus, dass sie pH stabil, lichtecht und in den Kapselölen gut löslich sind. Nach Belichtung in CB-Blatt weisen sie eine geringe Abnahme der Farbstärke (CB-Desaktivierung) auf.

Ein druckempfindliches Material besteht beispielsweise aus mindestens einem Paar von Blättern, die mindestens einen Farbbildner der Formeln (1) bis (3) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminiumphosphat, hydratisiertes Zirkondioxid, Zinkchlorid, Zinknitrat, aktiviertes Kaolin oder irgendein beliebiger Ton. Als Entwickler können auch sauer reagierende, organische Verbindungen, wie z.B. gegebenenfalls ringsbustituierte Phenole, Resorcine, Salicylsäuren, wie z.B. 3,5-Bis-($\alpha$,$\alpha$-dimethylbenzyl-)-salicylsäure oder 3,5-Bis-($\alpha$-methylbenzyl)-

salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein.

Die Entwickler können zusätzlich auch im Gemisch mit an sich unreaktiven oder wenig reaktiven Pigmenten oder weiteren Hilfstoffen wie Kieselgel oder UV-Absorbern, wie z.B. 2-(2'-Hydroxyphenyl-)benztriazolen eingesetzt werden. Beispiele für solche Pigmente sind: Talk, Titandioxid, Aluminiumoxid, Aluminiumhydroxyd, Zinkoxid, Kreide, Tone wie Kaolin, sowie organische Pigmente, z.B. Harnstoff-Formaldehydkondensate (BET-Oberfläche 2-75 $m^2/g$) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. halogeniertes Paraffin oder Diphenyl, wie Chlorparaffin, Monochlordiphenyl oder Trichlordiphenyl, ferner Tricresylphosphat, Di-n-butylphthalat, Dioctylphthalat, Trichlorbenzol, Trichlorethylphosphat, aromatische Ether, wie Benzylphenylether, Kohlenwasserstofföle, wie Paraffin oder Kerosin, z.B. mit Isopropyl, Isobutyl, sek-Butyl oder tert.-Butyl alkylierte Derivate von Diphenyl, Naphthalin oder Terphenyl, Dibenzyltoluol, partiell hydriertes Terphenyl, mono- bis tetra-$C_1$-$C_3$-alkylierte Diphenylalkane, Dodecylbenzol, benzylierte Xylole, oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen.

Die Kapselwände können durch Koazervationskräfte gleichmässig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (1) bis (3) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Vorzugsweise besteht das Durchschreibematerial auch darin, dass es eine kapselfreie, die Laktonverbindung enthaltende Schicht und eine farbentwickelnde Schicht, die als Farbentwickler mindestens ein anorganisches Metallsalz vor allem Halogenide oder Nitrate eines mehrwertigen Metalles, wie z.B. Zinkchlorid, Zinnchlorid, Zinknitrat oder deren Gemische enthält, aufweist.

Die Verbindungen der Formeln (1) bis (3) können auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel und/oder Wachs. Gewünschtenfalls können auch Aktivatoren oder Sensibilisatoren im Aufzeichnungsmaterial vorhanden sein.

Thermoreaktive Aufzeichnungssysteme umfassen, z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 12,51,348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäure-methylester oder -benzylester, 4-Hydroxydiphenylsulfon, 2,4-Dihydroxydiphenylsulfon, 4'-Hydroxy-4-methyldiphenylsulfon, 4'-Hydroxy-4-isopropoxydiphenylsulfon, 4-Hydroxy-acetophenon, 2,2'-Dihydroxydiphenyl, 4,4'-Cyclohexylidendiphenol, 4,4'-Isopropylidendiphenol, 4,4'-Isopropyliden-bis-(2-methylphenol), ein Antipyrinkomplex von Zinkthiocyanat, ein Pyridinkomplex von Zinkthiocyanat, 4,4-Bis-(4-hydroxyphenyl)valeriansäure, Hydrochinon, Pyrogallol, Phloroglucin, p-, m-, o-Hydroxybenzoesäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Laktonverbindungen und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, carboxylierte Butadien-Styrolcopolymerisate, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxyd, Calciumcarbonat (z.B. Kreide), Tone oder auch organische Pigmente, wie z.B. Harnstoff-Formaldehydpolymerisate enthalten. Um zu bewirken, dass nur innnerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Bis-Stearoyl-ethylendiamid, Stearinsäureamid, Phthalsäureanhydrid, Metallstearate, wie z.B. Zinkstearat, Phthalsäurenitril, Dimethylterephthalat, Dibenzylterephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanwachs, Paraffinwachs, Polyethylenwachs, Kondensate höherer Fettsäureamide und Formaldehyde und

Kondensate höherer Fettsäuren und Ethylendiamin.

Eine weitere Anwendung der Verbindungen der Formeln (1) bis (3) ist die Herstellung eines Farbbildes mittels photohärtbarer Mikrokapseln, wie sie z.B. in der DE-OS 3 247 488 beschrieben sind.

In den folgenden Beispielen beziehen sich die angegebenen Prozentsätze, wenn nichts anderes angegeben ist, auf das Gewicht. Teile bedeuten Gewichtsteile.

Beispiel 1

15,7 g 2-Hydroxy-4-diethylamino-2'-carboxy-benzophenon werden unter Rühren in 50 ml Schwefelsäure (98 %) bei Raumtemperatur gelöst. In diese Lösung werden portionenweise 8,7 g 1-Phenyl-5-methyl-pyrazol-(3)-on eingetragen, worauf das Gemisch über Nacht (10-12 Stunden) bei Raumtemperatur gerührt wird. Man giesst das Reaktionsgemisch auf Eiswasser und stellt mit konz. Ammoniakwasser alkalisch ein. Das ausgeschiedene Produkt wird abfiltriert, mit Wasser gewaschen und bei 60-70 °C im Vakuum getrocknet. Man erhält 23 g eines Rohproduktes, das nach Umkristallisation aus Ligroin/Toluol 15,2 g einer Verbindung der Formel

(11) 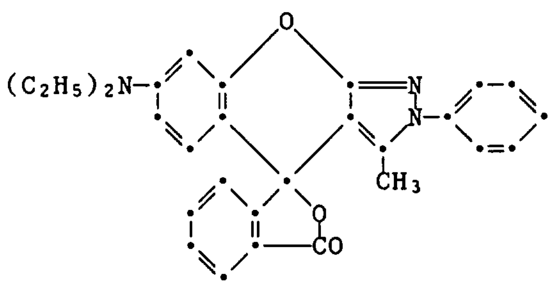

mit einem Schmelzpunkt von 235-237 °C ergibt. Auf Säureton erzeugt diese Laktonverbindung sofort eine intensive und lichtechte orange Färbung.

Verwendet man in Beispiel 1 anstelle von 2-Hydroxy-4-diethylamino-2'-carboxy-benzophenon die äquivalente Menge von

a) 2-Hydroxy-4-diethylamino-2'-carboxy-3',4',5',6'-tetrachlor-benzophenon oder

b) 2-Hydroxy-4-diethylamino-2'-carboxy-4'/5'-tert.butyl-benzophenon.

und verfährt wie im Beispiel beschrieben, so werden die Laktonverbindungen der Formeln (12) und (13) erhalten, welche auf saurem Ton eine intensive orange Farbe erzeugen.

(12)

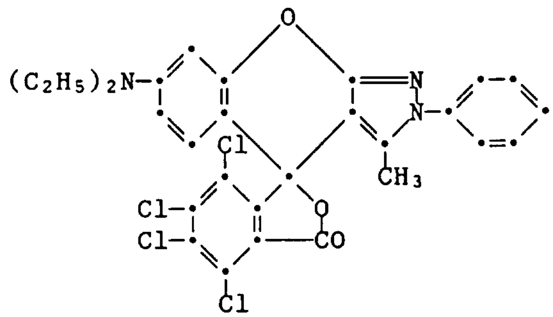

Smp. 241-242°C

(13)

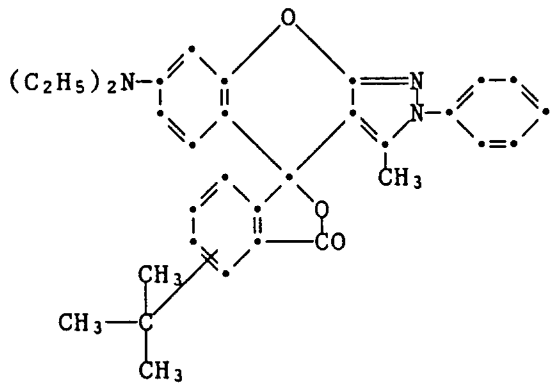

Smp. 117-137°C

Auf gleiche Weise wie in Beispiel 1 beschrieben, erhält man unter Verwendung der entsprechenden Ausgangsstoffe die in der folgenden Tabelle aufgeführten Laktonverbindungen der Formel

(14)

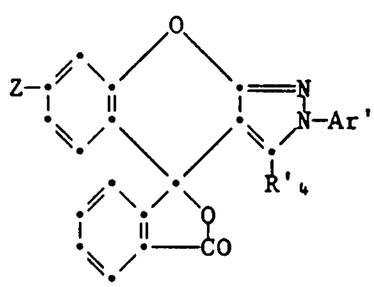

welche auf saurem Ton die angegebene Farbe ergeben.

Tabelle

| Bsp. | Z | Ar' | R'$_4$ | Smp/°C | Farbe |
|---|---|---|---|---|---|
| 2 | $-N(C_2H_5)_2$ | 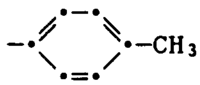 | $-CH_3$ | 113-125 | orange |
| 3 | $-N(C_2H_5)_2$ | 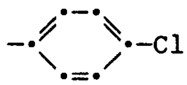 | $-CH_3$ | 115-128 | orange |
| 4 | $-N(C_4H_9)_2$ | 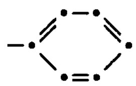 | $-CH_3$ | 87-90 | orange |
| 5 | $-N(C_2H_5)_2$ | 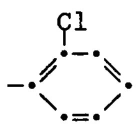 | $-CH_3$ | 195-202 | gelb |
| 6 | $-N(C_2H_5)_2$ | 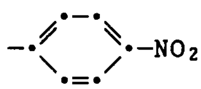 | $-CH_3$ | 198-200 | rot |
| 7 | $-N(C_2H_5)_2$ | 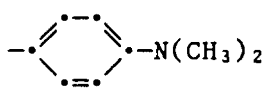 | $-CH_3$ | 131-145 | rot |
| 8 | $-N(C_2H_5)_2$ | 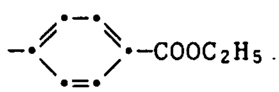 | $-CH_3$ | 110-115 | orange |
| 9 | $-N(C_2H_5)_2$ | 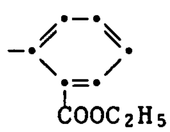 | $-CH_3$ | 110-120 | orange |
| 10 | $-N(C_2H_5)_2$ | 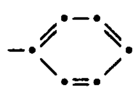 | 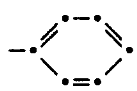 | 120-125 | orange |
| 11 | 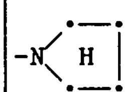 | 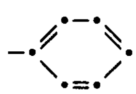 | $-CH_3$ | 150-160 | orange |

Beispiel 12: Eine Suspension von 4,7 g der gemäss Beispiel 6 hergestellten Benzopyrano-2H-pyrazolverbindung der Formel

(15) 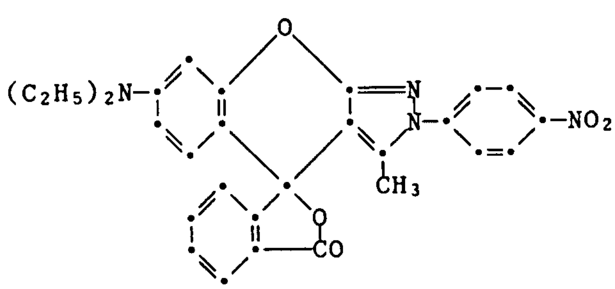

in 9,2 ml Wasser und 27,4 ml konz. Salzsäure wird unter Rühren auf 70-80 °C erwärmt. Hierauf werden portionenweise 6,2 g Zinndichlorid-2-Hydrat zugegeben unter Erhöhung der Temperatur auf 90-92 °C. Nach zwei Stunden ist die Reduktion der Nitroverbindung beendet. Man verdünnt mit 150 ml Wasser und stellt das Reaktionsprodukt mit konz. Natriumhydroxidlösung auf pH 12 ein. Man rührt weiter 30 Minuten bei 70-80 °C und filtriert das Produkt ab. Nach Waschen und Trocknen bei 80 °C im Vakuum erhält man 4,4 g einer Verbindung der Formel

(16)

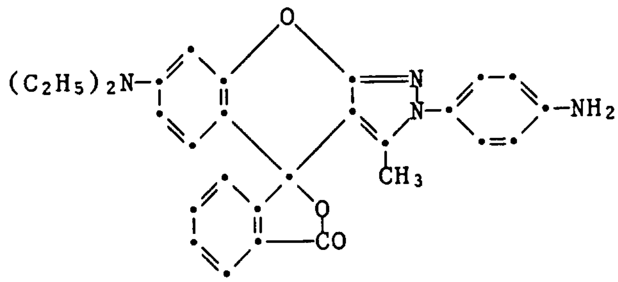

mit einem Schmelzpunkt von 255-260 °C.

a) 1,3 g der Verbindung der Formel (16) werden in 10 ml Acetanhydrid auf 100 °C erhitzt, dann mit Wasser versetzt und gerührt. Das ausgefallene Produkt wird abfiltriert, mit Wasser gewaschen und im Vakuum bei 70 °C getrocknet. Nach Reinigung über die Kieselgelsäule mit Methylendichlorid erhält man 0,8 g einer Verbindung der Formel

(17)

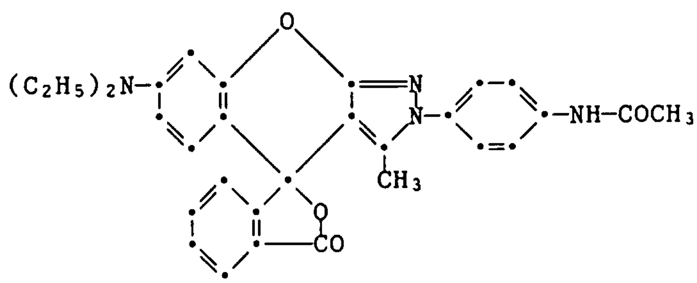

mit einem Schmelzpunkt von 176-182 °C. Auf Säureton entwickelt diese Laktonverbindung eine intensive orange Färbung.

b) 1,3 g der Verbindung der Formel (16) werden in 10 ml Benzylchlorid unter Rühren 10 Stunden bei 150-160 °C erhitzt, worauf überschüssiges Benzylchlorid durch Abdampfen entfernt wird. Der Rückstand wird mit verdünnter Ammoniaklösung behandelt und abfiltriert. Nach Waschen, Trocknen im Vakuum bei 60-70 °C und Reinigung über Kieselgelsäule mit Methylendichlorid erhält man eine Verbindung der Formel

(18)

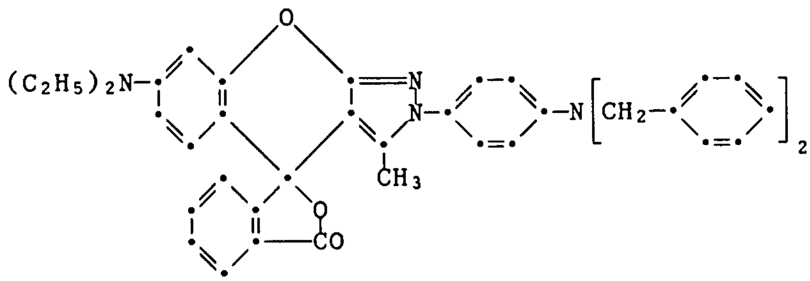

mit einem Schmelzpunkt von 188-200 °C. Auf Säureton entwickelt diese Laktonverbindung eine intensive purpurrote Färbung.

Beispiel 13: Herstellung eines druckempfindlichen Kopierpapiers

Eine Lösung von 3 g der Laktonverbindung der Formel (11) in 80 g Di-Isopropylnaphthalin und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit säureaktiviertem Bentonit als Farbentwickler beschichtet. Das erste, den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive orange Kopie, die ausgezeichnet sublimier- und lichtecht ist.

Entsprechende intensive, sublimier- und lichtechte Kopien werden auch bei Verwendung der Farbbildner gemäss den Beispielen 2 bis 12 erzielt.

Beispiel 14: Ersetzt man in Beispiel 13 die Laktonverbindung der Formel (11) durch eine Mischung der folgenden Zusammensetzung
1,2 g 3,3-Bis-(4'-dimethylaminophenyl)-6-dimethylaminophthalid,
1,2 g N-Butylcarbazol-3-yl-bis-(4'-N-methyl-N-phenylaminophenyl-)-methan
1,2 g der Laktonverbindung der Formel (11) und
0,4 g 3,3-Bis-(N-octyl-2'-methylindol-3'-yl-)phthalid
und verfährt im übrigen wie in Beispiel 13 beschrieben, so erhält man ein druckempfindliches Aufzeichnungsmaterial, welches durch Schreiben mit der Hand oder mit der Schreibmaschine eine intensive und lichtechte schwarze Kopie ergibt.

Beispiel 15: 1 g der Laktonverbindung gemäss Beispiel 6 wird in 17 g Toluol gelöst. Zu dieser Lösung gibt man unter Rühren 12 g Polyvinylacetat, 8 g Calciumcarbonat und 2 g Titandioxyd. Die erhaltene Suspension wird im Gewichtsverhältnis 1:1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 $g/m^2$ mit einer Mischung bestehend aus 1 Teil eines Amidwachses, 1 Teil eines Stearinwachses und 1 Teil Zinkchlorid beschichtet worden ist. Durch Schreiben mit der Hand oder mit der Schreibmaschine auf dem oberen Blatt wird Druck ausgeübt, und es entwickelt sich sofort auf dem mit den Farbbildner beschichteten Blatt, eine intensive, sublimier- und lichtechte rote Farbe.

Beispiel 16: Herstellung eines wärmeempfindlichen Aufzeichnungsmaterials

In einer Kugelmühle werden 32 g 4,4'-Isopropylidendiphenol (Bisphenol A), 3,8 g Distearylamid des Ethylendiamins, 39 g Kaolin, 20 g eines 88 % hydrolysierten Polyvinylalkohols und 500 ml Wasser gemahlen bis die Teilchengrösse ca 5 µm beträgt. In einer zweiten Kugelmühle werden 6 g der Laktonverbindung der Formel (11), 3 g eines zu 88 % hydrolysierten Polyvinylalkohols und 60 ml Wasser zu einer Teilchengrösse von ca. 3 µm gemahlen.

Die beiden Dispersionen werden zusammengegeben und mit einem Trockenauftragsgewicht von 5,5 $g/m^2$ auf ein Papier gestrichen. Durch Berührung des Papiers mit einem erhitzten Metallstift wird eine intensive orange Farbe erhalten, die eine ausgezeichnete Sublimier- und Lichtechtheit hat.

Intensive und lichtechte Farben werden auch bei Verwendung der Farbbildner gemäss den Beispielen 2 bis 12 erhalten.

## Patentansprüche

**1.** Chromogene Laktonverbindungen von Benzopyrano-2H-pyrazolen der Formel

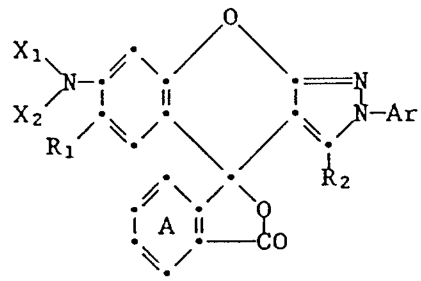

(1)

worin

| | |
|---|---|
| Ar | einen unsubstituierten oder durch Halogen, Nitro, Cyano, $C_1$-$C_5$-Alkyl, $C_1$-$C_5$-Alkoxy, $C_1$-$C_5$-Alkylthio, $C_1$-$C_5$-Alkoxycarbonyl, Trifluormethyl, Phenoxy, Phenylthio oder $-NX_3X_4$ substituierten Diphenyl-, Naphthyl- oder Phenylrest, |
| $R_1$ | Wasserstoff, Halogen, $C_1$-$C_5$-Alkyl oder $C_1$-$C_5$-Alkoxy, |
| $R_2$ | $C_1$-$C_5$-Alkyl, Phenyl oder durch Halogen, $C_1$-$C_5$-Alkyl oder $C_1$-$C_5$-Alkoxy substituiertes Phenyl, und |
| $X_1$, $X_2$, $X_3$ und $X_4$, | unabhängig voneinander, je Wasserstoff, unsubstituiertes oder durch Halogen, Hydroxy, Cyano, Tetrahydrofuryl oder $C_1$-$C_5$-Alkoxy substituiertes Alkyl mit höchstens 12 Kohlenstoffatomen, Acyl mit 1 bis 12 Kohlenstoffatomen, Cycloalkyl mit 5 bis 10 Kohlenstoffatomen oder unsubstituiertes oder durch Halogen, Cyano, Nitro, Trifluormethyl, $C_1$-$C_5$-Alkyl, $C_1$-$C_5$-Alkoxy oder $C_1$-$C_5$-Alkoxycarbonyl substituiertes Phenethyl, Phenylisopropyl oder Benzyl oder einen Diphenyl-, Naphthyl- oder Phenylrest, oder die Substituentenpaare ($X_1$ und $X_2$) und ($X_3$ und $X_4$) je zusammen mit dem gemeinsamen Stickstoffatom einen fünf- oder sechsgliedrigen Pyrrolidino-, Piperidino-, Pipecolino-, Morpholino-, Thiomorpholino- oder Piperazinorest bedeuten und worin |

der Ring A unsubstituiert oder durch Halogen, Nitro, $C_1$-$C_5$-Alkyl, $C_1$-$C_5$-Alkoxy, $C_1$-$C_5$-Alkylthio, $C_1$-$C_5$-Alkoxycarbonyl, Amino, Mono-$C_1$-$C_5$-Alkylamino oder Di-$C_1$-$C_5$-Alkylamino substituiert ist.

2. Laktonverbindungen gemäss Anspruch 1, dadurch gekennzeichnet, dass in Formel (1) $R_1$ Wasserstoff, Brom, Chlor, Methyl oder Methoxy bedeutet.

3. Laktonverbindungen gemäss einem der Ansprüche 1 und 2, dadurch gekennzeichnet, dass in Formel (1) $R_2$ $C_1$-$C_5$-Alkyl bedeutet.

4. Laktonverbindungen gemäss einem der Ansprüche 1 bis 3, dadurch gekennzeichnet, dass in Formel (1) Ar Phenyl, Naphthyl oder durch Halogen, Nitro, $C_1$-$C_5$lkyl, $C_1$-$C_5$-Alkoxy, $C_1$-$C_5$-Alkoxycarbonyl, Phenoxy oder $-NX_3X_4$ substituiertes Phenyl oder Naphthyl bedeutet.

5. Laktonverbindungen gemäss einem der Ansprüche 1 bis 4, dadurch gekennzeichnet, dass in Formel (1) $X_1$ und $X_2$ je $C_1$-$C_5$-Alkyl, Cyclohexyl, Tolyl, Benzyl oder Cyano-$C_1$-$C_5$-Alkyl bedeuten.

6. Laktonverbindungen gemäss einem der Ansprüche 1 bis 5, dadurch gekennzeichnet, dass in Formel (1) der Ring A unsubstituiert oder durch Halogen substituiert ist.

7. Laktonverbindungen gemäss Anspruch 1, dadurch gekennzeichnet, dass sie der Formel

(2)

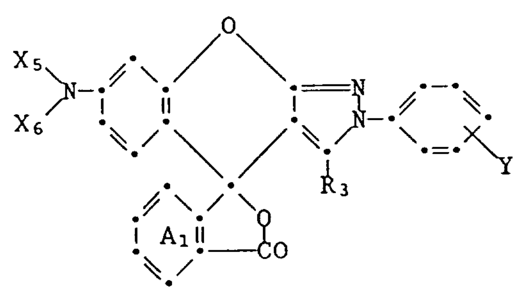

entsprechen,
worin

| | |
|---|---|
| $R_3$ | $C_1$-$C_5$-Alkyl oder Phenyl, |
| Y | Wasserstoff, Halogen, Nitro, $C_1$-$C_5$-Alkyl, $C_1$-$C_5$-Alkoxy, Phenoxy oder $-NX_7X_8$, |
| $X_8$, $X_8$, $X_7$ und $X_8$, | unabhängig voneinander, je $C_1$-$C_8$-Alkyl, $C_5$-$C_6$-Cycloalkyl oder unsubstituiertes oder durch Halogen, $C_1$-$C_5$-Alkyl, $C_1$-$C_5$-Alkoxy oder $C_1$-$C_5$-Alkyoxycarbonyl substituiertes Benzyl oder Phenyl und $X_8$ auch Wasserstoff oder |

die Substituentenpaare ($X_8$ und $X_8$) und ($X_7$ und $X_8$), unabhängig voneinander, je zusammen mit dem

jeweiligen gemeinsamen Stickstoffatom Pyrrolidino, Piperidino oder Morpholino bedeuten und der Ring $A_1$ unsubstituiert oder durch Halogen, $C_1$-$C_5$lkyl, $C_1$-$C_5$-Alkoxycarbonyl oder Di-$C_1$-$C_5$-Alkylamino substituiert ist.

**8.** Laktonverbindungen gemäss Anspruch 7, dadurch gekennzeichnet, dass in Formel (2)
$R_3$ $C_1$-$C_5$-Alkyl, und
Y Wasserstoff, Halogen, Nitro, Methyl oder Di-$C_1$-$C_5$-Alkylamino bedeuten und der Ring $A_1$ unsubstituiert ist.

**9.** Laktonverbindungen gemäss Anspruch 1, dadurch gekennzeichnet, dass sie der Formel

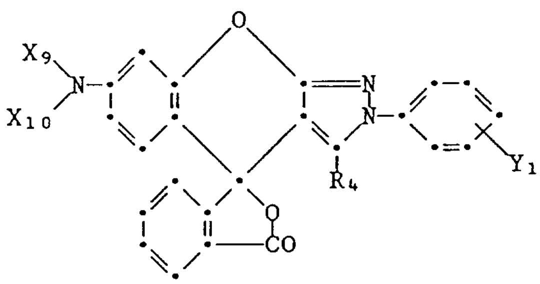

(3)

entsprechen,
worin

| | |
|---|---|
| $R_4$ | Methyl oder Phenyl, |
| $Y_1$ | Wasserstoff, Chlor, Nitro, Methyl, Carbomethoxy oder Di-$C_1$-$C_5$-Alkylamino, |
| $X_9$ | $C_1$-$C_6$-Alkyl, Cyclohexyl, Benzyl, Phenyl oder durch Methyl oder Chlor substituiertes Phenyl und |
| $X_{10}$ | $C_1$-$C_6$-Alkyl bedeuten. |

**10.** Laktonverbindungen gemäss Anspruch 9, dadurch gekennzeichnet, dass in Formel (3) $X_9$ und $X_{10}$ $C_1$-$C_5$-Alkyl, $R_4$ Methyl und $Y_1$ Wasserstoff, Chlor, Nitro, Methyl oder Dimethylamino bedeuten.

**11.** Die Laktonverbindung gemäss Anspruch 10, worin $X_9$ und $X_{10}$ Ethyl und $Y_1$ Wasserstoff bedeuten.

**12.** Verfahren zur Herstellung von Laktonverbindungen von Benzopyrano-2H-pyrazolen gemäss Anspruch 1, dadurch gekennzeichnet, dass man eine Ketosäureverbindung der Formel

(4)

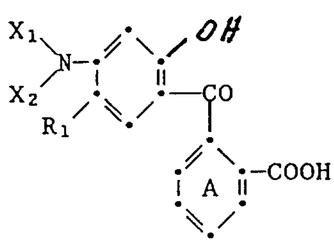

mit einer Pyrazolonverbindung der Formel

(5)

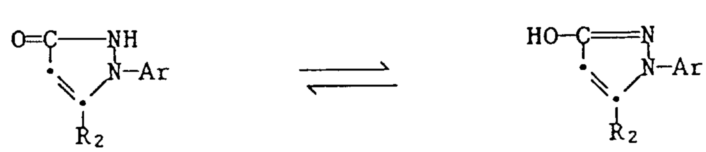

worin A, Ar, $R_1$, $R_2$, $X_1$ und $X_2$ die in Anspruch 1 angegebene Bedeutung haben, umsetzt.

**13.** Druck- oder wärmeempfindliches Aufzeichnungsmaterial, dadurch gekennzeichnet, dass es in seinem Farbreaktantensystem als Farbbildner mindestens eine Laktonverbindung gemäss einem der Ansprüche 1 bis 11 enthält.

**14.** Druckempfindliches Aufzeichnungsmaterial gemäss Anspruch 13, dadurch gekennzeichnet, dass es die Laktonverbindung, gelöst in einem organischen Lösungsmittel, und mindestens einen festen Elektronenakzeptor enthält.

**15.** Druckempfindliches Aufzeichnungsmaterial gemäss einem der Ansprüche 13 und 14, dadurch gekennzeichnet, dass die Laktonverbindung in Mikrokapseln eingekapselt ist.

**16.** Druckempfindliches Aufzeichnungsmaterial gemäss Anspruch 15, dadurch gekennzeichnet, dass die eingekapselte Laktonverbindung in Form einer Schicht auf der Rückseite eines Uebertragungsblattes und der Elektronenakzeptor in Form einer Schicht auf der Vorderseite des Empfangsblattes vorhanden sind.

**17.** Wärmeempfindliches Aufzeichnungsmaterial gemäss Anspruch 13, dadurch gekennzeichnet, dass es in mindestens einer Schicht mindestens eine Laktonverbindung gemäss einem der Ansprüche 1 bis 11, einen Elektronenakzeptor und gegebenenfalls ein Bindemittel und/oder Wachs enthält.

**18.** Druckempfindliches oder wärmeempfindliches Aufzeichnungsmaterial gemäss einem der Ansprüche 13 bis 17, dadurch gekennzeichnet, dass die Laktonverbindung gemeinsam mit einem oder mehreren anderen Farbbildnern enthalten ist.

## Claims

**1.** A chromogenic benzopyrano-2H-pyrazole lactone compound of the formula

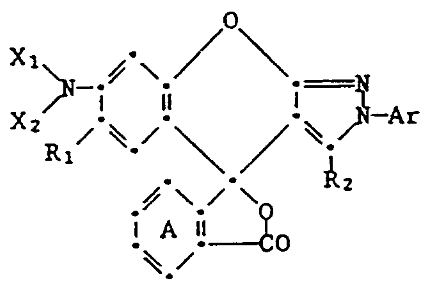

(1)

in which

Ar is a biphenylyl, naphthyl or phenyl radical which is unsubstituted or substituted by halogen, nitro, cyano, $C_1$-$C_5$alkyl, $C_1$-$C_5$alkoxy, $C_1$-$C_5$alkylthio, $C_1$-$C_5$alkoxycarbonyl, trifluoromethyl, phenoxy, phenylthio or $-NX_3X_4$,

$R_1$ is hydrogen, halogen, $C_1$-$C_5$alkyl or $C_1$-$C_5$alkoxy,

$R_2$ is $C_1$-$C_5$alkyl, phenyl, or phenyl which is substituted by halogen $C_1$-$C_5$alkyl or $C_1$-$C_5$alkoxy, and

$X_1$, $X_2$, $X_3$ and $X_4$, independently of one another, are each hydrogen, alkyl having a maximum of 12 carbon atoms which is unsubstituted or substituted by halogen, hydroxyl, cyano, tetrahydrofuryl or $C_1$-$C_5$alkoxy, or are acyl having 1 to 12 carbon atoms, cycloalkyl having 5 to 10 carbon atoms, or phenethyl, phenylisopropyl or benzyl, each of which is unsubstituted or substituted by halogen, cyano, nitro, trifluoromethyl, $C_1$-$C_5$alkyl, $C_1$-$C_5$alkoxy or $C_1$-$C_5$alkoxycarbonyl, or a biphenylyl, naphthyl or phenyl radical, or the substituent pairs ($X_1$ and $X_2$) and ($X_3$ and $X_4$), in each case together with the common nitrogen atom, are a five- or six-membered heterocyclic radical, and in which

the ring A is unsubstituted or substituted by halogen, nitro, $C_1$-$C_5$alkyl, $C_1$-$C_5$alkoxy, $C_1$-$C_5$alkylthio, $C_1$-$C_5$alkoxycarbonyl, amino, mono ($C_1$-$C_5$alkyl)amino or di($C_1$-$C_5$alkyl)amino.

**2.** A lactone compound according to claim 1, wherein in the formula (1), $R_1$ is hydrogen, bromine, chlorine, methyl or methoxy.

**3.** A lactone compound according to either of claims 1 and 2, wherein, in the formula (1), $R_2$ is $C_1$-$C_5$alkyl.

**4.** A lactone compound according to any one of claims 1 to 3, wherein, in the formula (1), Ar is phenyl, naphthyl or phenyl or naphthyl which is substituted by halogen, nitro, $C_1$-$C_5$alkyl, $C_1$-$C_5$alkoxy, $C_1$-$C_5$alkoxycarbonyl, phenoxy or -$NX_3X_4$.

**5.** A lactone compound according to any one of claims 1 to 4, wherein, in the formula (1), $X_1$ and $X_2$ are each $C_1$-$C_5$alkyl, cyclohexyl, tolyl, benzyl or cyano($C_1$-$C_5$alkyl).

**6.** A lactone compound according to any one of claims 1 to 5, wherein, in the formula (1), the ring $A_1$ is unsubstituted or substituted by halogen.

**7.** A lactone compound according to claim 1, conforming to the formula

(2) 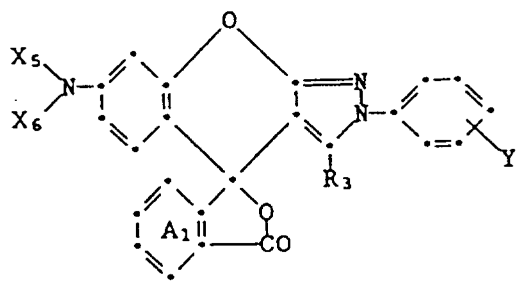

in which
$R_3$ is $C_1$-$C_5$alkyl or phenyl,
Y is hydrogen, halogen, nitro, $C_1$-$C_5$alkyl, $C_1$-$C_5$alkoxy phenoxy or -$NX_7X_8$, $X_8$, $X_8$, $X_7$ and $X_8$, independently of one another, are each $C_1$-$C_8$alkyl, $C_5$-$C_6$cycloalkyl or benzyl or phenyl, each of which is unsubstituted or substituted by halogen, $C_1$-$C_5$alkyl, $C_1$-$C_5$alkoxy or $C_1$-$C_5$alkoxycarbonyl, and $X_8$ is alternatively hydrogen, or the substituent pairs ($X_8$ and $X_8$) and ($X_7$ and $X_8$), independently of one another and in each case together with the respective common nitrogen atom, are pyrrolidino, piperidino or morpholino, and the ring $A_1$ is unsubstituted or substituted by halogen, $C_1$-$C_5$alkyl, $C_1$-$C_5$alkoxycarbonyl or di($C_1$-$C_5$alkyl)amino.

**8.** A lactone compound according to claim 7, wherein, in the formula (2),
$R_3$ is $C_1$-$C_5$alkyl, and
Y is hydrogen, halogen, nitro, methyl or di ($C_1$-$C_5$alkyl)amino, and the ring $A_1$ is unsubstituted.

**9.** A lactone compound according to claim 1, conforming to the formula

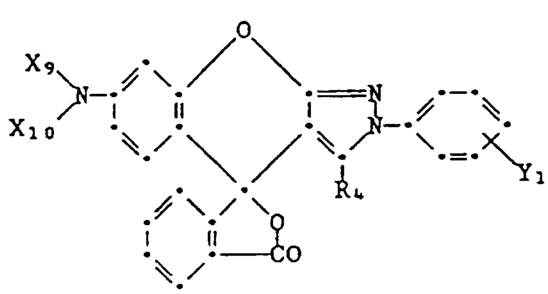 (3)

in which
$R_4$ is methyl or phenyl,
$Y_1$ is hydrogen, chlorine, nitro, methyl, carbomethoxy or di($C_1$-$C_5$alkyl)amino,

$X_9$ is $C_1$-$C_6$alkyl, cyclohexyl, benzyl, phenyl, or phenyl which is substituted by methyl or chlorine, and $X_{10}$ is $C_1$-$C_6$alkyl.

**10.** A lactone compound according to claim 9, wherein, in the formula (3), $X_9$ and $X_{10}$ are $C_1$-$C_5$alkyl, $R_4$ is methyl and $Y_1$ is hydrogen, chlorine, nitro, methyl or dimethylamino.

**11.** The lactone compound according to claim 10 in which $X_9$ and $X_{10}$ are ethyl and $Y_1$ is hydrogen.

**12.** A process for the preparation of a benzopyrano-2H-pyrazole lactone compound according to claim 1, wherein a keto acid compound of the formula

(4) 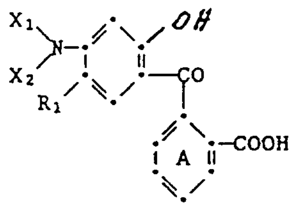

is reacted with a pyrazolone compound of the formula

(5) 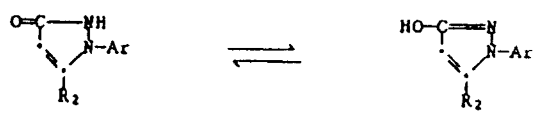

in which

A, Ar, $R_1$, $R_2$, $X_1$ and $X_2$ have the definition indicated in claim 1.

**13.** A pressure-sensitive or heat-sensitive recording material containing, as colour former, at least one lactone compound according to any one of claims 1 to 11, in its colour reactant system.

**14.** A pressure-sensitive recording material according to claim 13, containing the lactone compound, dissolved in an organic solvent, and at least one solid electron acceptor.

**15.** A pressure-sensitive recording material according to either of claims 13 and 14, wherein the lactone compound is encapsulated in microcapsules.

**16.** A pressure-sensitive recording material according to claim 15, wherein the encapsulated lactone compound is present in the form of a coating on the rear of a transfer sheet, and the electron acceptor is present in the form of a coating on the front of the receiver sheet.

**17.** A heat-sensitive recording material according to claim 13, containing, in at least one coating, at least one lactone compound according to any one of claims 1 to 11, an electron acceptor and, if appropriate, a binder and/or wax.

**18.** A pressure-sensitive or heat-sensitive recording material according to any one of claims 13 to 17, wherein the lactone compound is present together with one or more other colour formers.

## Revendications

**1.** Composés de type lactone de benzopyrano-2H-pyrazoles de formule

(1) 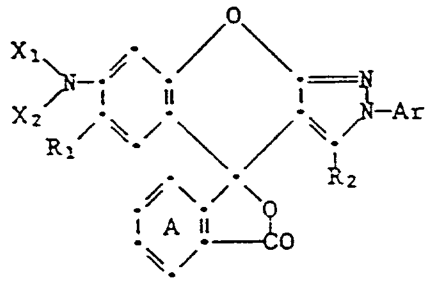

dans laquelle
Ar représente un résidu diphényle, naphtyle ou phényle, non substitué ou substitué par un ou des substituants halogéno, nitro, cyano, alkyle en $C_1$-$C_5$, alcoxy en $C_1$-$C_5$, alkylthio en $C_1$-$C_5$, alcoxy(en $C_1$-$C_5$)-carbonyle, trifluorométhyle, phénoxy, phénylthio ou $-NX_3X_4$,
$R_1$ représente un atome d'hydrogène ou d'halogène, un groupe alkyle en $C_1$-$C_5$ ou alcoxy en $C_1$-$C_5$,
$R_2$ représente un groupe alkyle en $C_1$-$C_5$, un groupe phényle substitué ou non par un atome d'halogène ou par un groupe alkyle en $C_1$-$C_5$ ou alcoxy en $C_1$-$C_5$, et
$X_1$, $X_2$, $X_3$ et $X_4$ représentent chacun, indépendamment l'un de l'autre, un atome d'hydrogène, un groupe alkyle comportant au plus 12 atomes de carbone et portant ou non un ou des substituants halogéno, hydroxy, cyano, tétrahydrofuryle ou alcoxy en $C_1$-$C_5$, un groupe acyle comportant de 1 à 12 atomes de carbone, un groupe cycloalkyle comportant de 5 à 10 atomes de carbone, ou un groupe phényléthyle, phénylisopropyle, benzoyle, diphényle, naphtyle ou phényle substitué ou non par un atome d'halogène, un groupe cyano, nitro, trifluorométhyle, alkyle en $C_1$-$C_5$, alcoxy en $C_1$-$C_5$ ou alcoxy(en $C_1$-$C_5$)-carbonyle, ou bien chacune des paires de substituants ($X_1$ et $X_2$) et ($X_3$ et $X_4$) peut constituer conjointement avec un atome d'azote en commun un groupe pyrrolidino, pipéridino, pipécolino, morpholino, thiomorpholino ou pipérazino à 5 ou 6 chaînons et de préférence saturé, et
le cycle A n'est pas substitué ou substitué par un atome d'halogène, un groupe nitro, alkyle en $C_1$-$C_5$, alcoxy en $C_1$-$C_5$, alkyltio en $C_1$-$C_5$, alcoxy(en $C_1$-$C_5$)-carbonyle, amino, monoalkyl(en $C_1$-$C_5$)-amino ou di(alkyle en $C_1$-$C_5$)-amino.

**2.** Composés de type lactone conformes à la revendication 1, caractérisés en ce que, dans la formule (1), $R_1$ représente un atome d'hydrogène, de brome ou de chlore ou un groupe méthyle ou méthoxy,.

**3.** Composés de type lactone conformes à une des revendications 1 et 2, caractérisés en ce que, dans la formule (1), $R_2$ représente un groupe alkyle en $C_1$-$C_5$.

**4.** Composés de type lactone conformes à une des revendications 1 à 3, caractérisés en ce que, dans la formule (1) Ar représente des groupes phényle ou naphtyle portant ou non un substituant halogéno, nitro, alkyle en $C_1$-$C_5$, alcoxy en $C_1$-$C_5$, alcoxy(en $C_1$-$C_5$)-carbonyle, phénoxy ou $-NX_3X_4$.

**5.** Composés de type lactone conformes à une des revendications 1 à 4, caractérisés en ce que, dans la formule (1), $X_1$ et $X_2$ représentent un groupe alkyle en $C_1$-$C_5$, cyclohexyle, tolyle, benzoyle ou cyanoalkyle en $C_1$-$C_5$.

**6.** Composés de type lactone conformes à une des revendications 1 à 5, caractérisés en ce que, dans la formule (1), le cycle A est non substitué ou halogéné.

**7.** Composés de type lactone conformes à la revendication 1, caractérisés en ce qu'ils correspondent à la formule

(2)

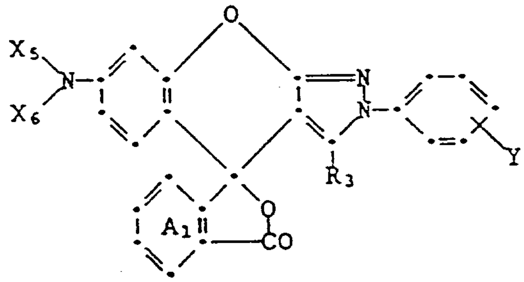

dans laquelle
$R_3$ représente un groupe alkyle en $C_1$-$C_5$ ou phényle,
Y représente un atome d'hydrogène ou d'halogène ou un groupe nitro, alkyle en $C_1$-$C_5$, alcoxy en $C_1$-$C_5$, phénoxy ou $-NX_7X_8$,
$X_8$, $X_8$, $X_7$ et $X_8$ représentent chacun, indépendamment l'un de l'autre, un groupe alkyle en $C_1$-$C_8$, cycloalkyle en $C_5$-$C_6$, ou des résidus benzyle ou phényle substitués ou non par un ou des atomes halogènes et groupes alkyle en $C_1$-$C_5$, alcoxy en $C_1$-$C_5$ ou alcoxy(en $C_1$-$C_5$)-carbonyle, et $X_8$ peut également représenter un atome d'hydrogène ou bien
les paires de substituants ($X_8$ et $X_8$) et ($X_7$ et $X_8$) peuvent constituer chacune conjointement avec l'atome d'azote en commun un groupe pyrrolidino, pipéridino ou morpholino,
et le cycle $A_1$ n'est pas substitué ou porte un ou des substituants halogéno, alkyle en $C_1$-$C_5$, alcoxy(en $C_1$-$C_5$)-carbonyle ou di(alkyle en $C_1$-$C_5$)-amino.

**8.** Composés de type lactones conformes à la revendication 7, caractérisés en ce que, dans la formule (2), $R_3$ représente un groupe alkyle en $C_1$-$C_5$, Y représente un atome d'hydrogène ou d'halogène ou un groupe nitro, méthyle ou di(alkyle en $C_1$-$C_5$)-amino et le cycle $A_1$ est non substitué.

**9.** Composés de type lactone conformes à la revendication 1, caractérisés en ce qu'ils correspondent à la formule

(3)

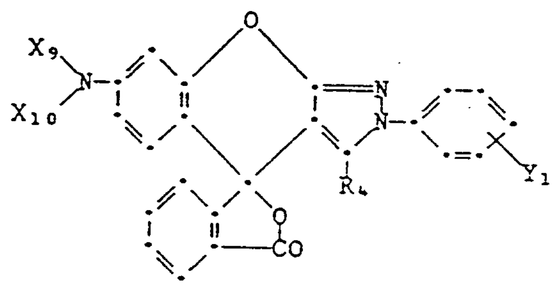

dans laquelle
$R_4$ représente un groupe méthyle ou phényle,
$Y_1$ représente un atome d'hydrogène ou de chlore ou un groupe nitro, méthyle, carbométhoxy ou di-(alkyle en $C_1$-$C_5$)-amino,
$X_9$ représente un groupe alkyle en $C_1$-$C_6$, cyclohexyle, benzyle ou phényle ou encore un groupe phényle portant un ou des substituants méthyle ou chlore, et
$X_{10}$ représente un groupe alkyle en $C_1$-$C_6$.

**10.** Composés de type lactone conformes à la revendication 9, caractérisés en ce que, dans la formule (3), $X_9$ et $X_{10}$ représentent un groupe alkyle en $C_1$-$C_5$, $R_4$ représente un groupe méthyle et $Y_1$ un atome d'hydrogène ou de chlore, un groupe nitro, méthyle ou diméthylamino.

**11.** Composés de type lactone conformes à la revendication 10, dans lesquels $X_9$ et $X_{10}$ représentent un groupe éthyle et $Y_1$ un atome d'hydrogène.

**12.** Procédé de préparation de composés de type lactone de benzopyrano-2H-pyrazoles conformes à la revendication 1, caractérisé en ce que l'on fait réagir un cétoacide de formule

(4) 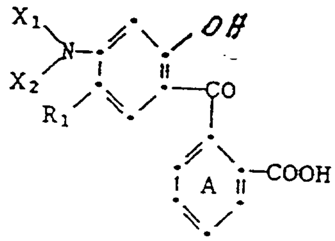

avec une pyrazolone de formule

(5) 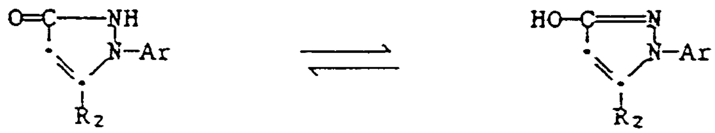

formules, dans lesquelles A, Ar, $R_1$, $R_2$, $X_1$ et $X_2$ ont la signification indiquée dans la revendication 1.

**13.** Matériau d'enregistrement sensible à la pression ou à la chaleur, caractérisé en ce qu'il contient, dans son système de réactifs chromogènes, comme agent chromogène au moins un composé de type lactone conforme à une des revendications 1 à 11.

**14.** Matériau d'enregistrement sensible à la pression conforme à la revendication 13, caractérisé en ce qu'il contient le composé de type lactone dissous dans un solvant organique et au moins un accepteur d'électrons solide.

**15.** Matériau d'enregistrement sensible à la pression conforme à une des revendications 13 et 14, caractérisé en ce que le composé de type lactone est encapsulé dans des microcapsules.

**16.** Matériau d'enregistrement sensible à la pression conforme à la revendication 15, caractérisé en ce que le composé de type lactone encapsulé se trouve sous forme d'une couche sur le côté verso d'une feuille de transfert et que l'accepteur d'électrons se trouve sous forme d'une couche sur le côté recto d'une feuille réceptrice.

**17.** Matériau d'enregistrement sensible à la chaleur conforme à la revendication 13, caractérisé en ce qu'il contient, dans au moins une couche, au moins un composé de type lactone conforme à une des revendications 1 à 11, un accepteur d'électrons et éventuellement un liant et/ou une cire.

**18.** Matériau d'enregistrement conforme à une des revendications 13 à 17, caractérisé en ce que le composé de type lactone est présent à côté d'un ou de plusieurs autres agents chromogènes.